# EUROPÄISCHE PATENTSCHRIFT

(11) **EP 1 231 947 B1**
(45) Veröffentlichungstag und Bekanntmachung des Hinweises auf die Patenterteilung: **24.03.2004**
(21) Anmeldenummer: 00988756.3
(22) Anmeldetag: 24.11.2000
(51) Int. Cl.: A61L 24/00, A61L 24/10, A61L 27/38, A61L 27/42, A61M 5/19, A61B 17/00

(54) **SPRITZBARES KNOCHENERSATZMATERIAL ENTHALTEND FIBRIN ODER FIBRINOGEN SOWIE HYDROXYLAPATIT ZEMENT**
INJECTABLE BONE-SUBSTITUTE MATERIAL COMPRISING FIBRIN OR FIBRINOGEN AND HYDROXYAPATITE CEMENT
MATERIAU SUBSTITUT OSSEUX INJECTABLE CONTENANT DE LA FIBRINE OU DU FIBROGENE ET DU CIMENT D'HYDROXYAPATITE

(30) Priorität: 24.11.1999 DE 19956503
(43) Veröffentlichungstag der Anmeldung: 21.08.2002
(73) Patentinhaber: Universitätsklinikum Freiburg, 79106 Freiburg (DE)
(72) Erfinder: SCHAEFER, Dirk, Johannes, 79102 Freiburg (DE); KIEFER, Thomas, 79117 Freiburg (DE); STARK, Gerhard, Björn, 79874 Breitnau (DE); KNESER, Ulrich c/o Universitätsklinikum Freiburg, 79106 Freiburg (DE)
(74) Vertreter: Keller, Günter, Dr.
(86) Internationale Anmeldenummer: PCT/EP2000/011716
(87) Internationale Veröffentlichungsnummer: WO 2001/037889

(56) Entgegenhaltungen:
- EP-A- 0 341 007
- WO-A-00/07639
- WO-A-95/26761
- WO-A-95/31137
- WO-A-98/40111
- WO-A-99/55252
- US-A- 5 914 121

## Beschreibung

Die vorliegende Erfindung betrifft ein Knochenersatzmaterial enthaltend lebende Zellen eine weiche Matrix und eine aushärtende Matrix. Die Erfindung betrifft ebenfalls Verfahren zur Herstellung eines derartigen Knochenersatzmaterials sowie die Verwendung von Hydroxylapatit-Zement zur Herstellung eines lebende Zellen enthaltenden Knochenersatzmaterials und deren Anwendung in einem geeigneten Spritzapparat.

Bei zahlreichen Knochendefekten ist es wünschenswert, ein Knochenersatzmaterial zur Verfügung zu haben, mit dem diese Defekte aufgefüllt werden können. Beispiele für derartige Defekte sind im Kieferbereich Parodontose oder Atrophien, im Handbereich Defekte nach Knochentumorresektionen und Traumata und Defekte der Wirbelsäule, des Schädels und der Röhrenknochen, beispielsweise bei Osteoporosefrakturen und Tumorresektionen.

Im Stand der Technik sind verschiedene Knochenersatzmaterialien bekannt. Knochenersatzmaterialien, die verformt werden können, werden oft als "injizierbarer Knochen" bezeichnet. Bisherige Lösungen unter diesem Begriff beinhalten entweder Hydrogele mit knochenbildenden Zellen, Hydrogele mit osteoinduktiven Proteinen oder Polymere, die sich in situ verfestigen, mit oder ohne osteoinduktiven Faktoren. Jede dieser Lösungen hat spezifische Nachteile.

Entweder enthalten die Materialien keine knochenbildenden Zellen, sie können also nicht osteogen wirken. In der Regel wird ein Biomaterial oder Knochenzement als homogene Plombe in einen Knochendefekt gespritzt, die nicht mehr resorbierbar und durch Knochen ersetzbar ist. Unter mechanischen Belastungen kommt es zur Ermüdung und zum Bruch des Implantats. Materialien, die Zellen enthalten, weisen zwar potentielle Osteogenität auf, besitzen aber keine Stabilität. Diese Materialien, meist in Form von Hydrogelen, können auch nicht geformt werden und haben keine Plastizität. Polymere, die in situ aushärten, wirken oft toxisch auf die Zellen. Die US-Patentschrift 5,914,121 offenbart eine Zusammensetzung zur Implantation in ein Säugetier umfassend Fibroblasten, Hydroxylapatit-Pulver und Fibrin. Diese Zusammensetzung weist keine Sekundärstabilität auf, weil sich das Material nach Implantation nicht verfestigt, sondern weiter verformbar ist. Grund dafür ist, daß keramisches Hydroxylapatit-Pulver verwendet wird. Diese Zusammensetzung härtet nicht aus, da die Partikel keine Verbindung untereinander eingehen, Im Stand der Technik gibt es kein Knochenersatzmaterial, das lebende Zellen enthält und somit osteogen wirken kann und zugleich ausreichende Sekundärstabilität bereitstellt.

Die US-PS 5 914 121 offenbart eine implantierbare, knochenbildende Zusammensetzung, enthaltend Knochenmark-Fibroblasten und Hydroxylapatit/Tricalcium-Phosphat-Pulver, die aber keine Sekundärstabilität aufweist, weil sich das Material nach Implantation nicht verfestigt.

Es besteht also ein dringendes Bedürfnis nach einem vorteilhaften Knochenersatzmaterial.

Die Aufgabe wurde gelöst durch ein Knochenersatzmaterial gemäß Anspruch 1. Das erfindungsgemäße Knochenersatzmaterial, das eine weiche Matrix, lebende Zellen und eine aushärtende Matrix umfaßt. Für das erfindungsgemäße Knochenersatzmaterial wurde eigens eine geeignete Misch- und Applikationseinheit entwickelt.

Das erfindungsgemäße Knochenersatzmaterial weist eine hervorragende Primär- und die Sekundärstabilität auf. Unter der Primärstabilität (auch "primäre Plastizität") eines Knochenersatzmaterials wird die Stabilität einer Zusammensetzung zum Zeitpunkt der Applikation verstanden. Das Knochenersatzmaterial der vorliegenden Erfindung ist plastisch verformbar und kann in konkrete dreidimensionale Formen gehracht werden, je nach den anatomischen Erfordernissen. Das Material ist also nicht zu "flüssig", da sich dann keine plastischen Gebilde würden formen lassen. Es ist aber auch nicht zu starr, im Extremfall sogar völlig erhärtet, da es dann nicht den Gegebenheiten des Falles einfach angepaßt werden könnte und da derartige "harte" Implantate in der Regel keine osteogenen Komponenten enthalten könnten. Unter Sekundärstabilität ist die Stabilität des Implantats nach dem Eingriff zu verstehen. Das Knochenersatzmaterial der Erfindung behält nach dem Aushärten langfristig die dreidimensionale Form, die ihm verliehen wurde. Es ist druckstabil. Dies wird dadurch erreicht, daß das erfindungsgemäße Material, das zuvor entsprechend geformt worden ist, in relativ kurzer Zeit vollständig aushärtet.

Die weiche Matrix gewährleistet das Überleben der Zellen, ihre Migration und Organisation in der Matrix und ihre Differenzierung zu knochenbildenden Osteoblasten. Eine weitere Funktion der weichen Matrix ist, daß sie zur Primärstabilität des Materials, also zur anfänglichen Formbarkeit, beiträgt. Die weiche Matrix ist vorzugsweise eine Fibrinsuspension, bevorzugter eine autogene oder allogene Fibrinsuspension, die aus einer Fibrinogenlösung hergestellt werden kann. Dies wird vorzugsweise durch Zugabe einer Thrombin-haltigen Lösung, bevorzugter einer auto- oder allogenen Thrombin-haltigen Lösung in Gegenwart von Calcium erreicht. Vor Zugabe des Thrombins kann die Fibrinogenlösung zur Stabilisierung des später entstehenden Fibrins durch ε-Aminocapronsäure, Aprotinin, Faktor 13 oder ähnliche Substanzen angereichert werden. Die weiche Matrix kann gegebenenfalls angereichert sein mit Chondroitinsulfat, Proteoglykanen, Sialoproteinen, Hormonen oder Wachstumsfaktoren. Beispiele für Wachstumsfaktoren, die zum Einsatz kommen können, sind bFGF, PDGF, VEGF, Bone Morphogenetic Proteins, TGF-β und weitere bekannte Faktoren. Nucleinsäuren, die die Wachstumsfaktoren oder Hormone kodieren, können ebenfalls in der weichen Matrix enthalten sein, vorzugsweise in Form von Plasmiden. Ergänzend können weitere viskose, gelierende und sich verfestigende Gele verwendet werden, wie zum Beispiel biologische Kollagengele, Gelatine, Alginate, Agarose, Polysaccharide, synthetisches Kollagen, Hydrogele oder visköse Polymere. Es können auch kommerzielle Fibrinkleber wie TissuCol® (Baxter) oder Beriplast (Aventis) eingesetzt werden, sie sind aber nicht bevorzugt.

Bei den lebenden Zellen des erfindungsgemäßen Knochenersatzmaterials handelt es sich vorzugsweise um Osteoblasten oder Vorläuferzellen von Osteoblasten. Diese können durch kleine Knochenbiopsien des Beckens, Brustbeins, des Schädels oder Kiefers oder von Röhrenknochen gewonnen werden. Alternativ zu Knochenproben können auch Aspirate von Knochenmark aus dem Becken und aus dem Brustbein verwendet werden. Gegebenenfalls können die gewonnenen Zellen in vitro kultiviert und vermehrt werden. Vorteilhafterweise enthält das Knochenersatzmaterial auch gefäßbildende Zellen, wie zum Beispiel Endothelzellen oder deren Vorläuferzellen.

Erfindungsgemäß handelt es sich bei den Zellen entweder um solche, die von dem Patienten selbst stammen (autologe Zellen) oder um Zellen bzw. Zellinien, die von dem Empfänger toleriert werden. Beispiele hierfür sind embryonale Stammzellen sowie allogene mesenchymale Stammzellen, Fibroblasten, stromale Zellen oder Osteoblasten, Endothelzellen, Muskelzellen, bzw. deren Vorläuferzellen. Ebenfalls können autogene mesenchymale Stammzellen, Fibroblasten, stromale Zellen oder Osteoblasten, Endothelzellen, Muskelzellen, bzw. deren Vorläuferzellen verwendet werden.

Das erfindungsgemäße Material umfaßt auch eine erhärtende Matrix. Dadurch verfestigt sich das Material innerhalb einer bestimmten Zeit zu einer stabilen Zusammensetzung. Bevorzugt verfestigt sich die Zusammensetzung innerhalb einer Stunde, am bevorzugtesten innerhalb von 15 Minuten. Das Knochenersatzmaterial weist eine pastöse Konsistenz auf, wodurch die Primärstabilität bereitgestellt wird. Dies bedeutet, daß das Material leicht an eine bestimmte Form angepaßt oder in eine bestimmte Form gebracht werden kann. Die Primärstabilität wird vorteilhafterweise durch die in der Zusammensetzung enthaltenen Fibrinstränge bereitgestellt. Die erhärtende Matrix ist für die Sekundärstabilität verantwortlich. Das bedeutet, daß die Zusammensetzung nach dem Erhärten nicht mehr verformbar ist, sondern Druckfestigkeit aufweist.

Vorzugsweise verbindet sich die erhärtende Matrix durch Kristallisation zu Hydroxylapatit. Die feste Matrix kann durch anorganische Verbindungen z.B. aus kristallinen oder amorphen Calciumphosphaten (Tetracalciumphosphat, α- oder β-Tricalciumphosphat, Dicalciumphosphat oder Dicalciumphosphatdihydrat) hergestellt werden. Vorzugsweise finden fertige sog. nicht-keramische Knochenzemente aus Kombinationen dieser Calciumphosphatverbindungen Anwendung (z.B. BoneSource®, Fa. Leibinger; Norian SRS®, Fa. Synthes-Stratec, USA; Biobone®, Fa. Merck, Darmstadt). Diese zeichnen sich dadurch aus, daß sie eine röntgenspektrometrische Diffraktion ähnlich dem der mineralischen Phase des Knochens haben, sich endothermal oder isothermal bei Körpertemperatur von 37°C in 10-15 Minuten zu mikroporösem Calciumphosphatzement durch Kristallisation verbinden, injizierbar sind, eine Druckstabilität (ca. 60 Mpa) größer oder gleich der von normalem Knochen aufweisen, chemische Verbindungen zum Empfängerknochen eingehen und als osteokonduktive Leitschiene fungieren können.

Calciumphosphatzement wird in vivo langsam resorbiert (ca. 35% in der ersten 12 Monaten). Das bevorzugteste Material der sich erhärtenden Matrix ist nicht-keramischer Hydroxylapatit-Zement. Einige Eigenschaften von Hydroxylapatit-Zement sind in Costantino P. D. et al. (1991) Archives of Otolaryngology - Head and Neck Surgery 117, 379 angegeben. Hydroxylapatit-Zement weist wesentliche Unterschiede zu sogenanntem keramischem Hydroxylapatit auf, welcher häufig in der klinischen Praxis verwendet wird. Hydroxylapatit-Zement verbindet sich erst durch direkte Kristallisation zu Hydroxylapatit. Die Bestandteile von Hydroxylapatit-Zement reagieren in wäßriger Umgebung zu Hydroxylapatit. Unter in vitro-Bedingungen bei 37° bindet reiner Hydroxylapatit-Zement in etwa 15 Minuten ab. Hydroxylapatit-Zement im Sinne dieser Anmeldung ist ein Calciumphosphat-Zement, der sich durch einen Kristallisationsprozeß zu Hydroxylapatit verbindet.

Um die Spritzbarkeit bzw. die mechanischen Eigenschaften zu verändern, können den Calciumphosphaten weitere Verbindungen zugegeben werden, wie z. B. Natriumchlorid-Lösung, Milchsäure, Glycerol, Chitosan, Natriumglycerolphosphat, Propylenfumarat oder bioaktive Proteine.

Auch andere Materialien, wie Poly-Glykol-Milchsäure (Polyglycolic-lactid-acid, PGLA), können ebenfalls eingesetzt werden.

Die vorliegende Erfindung stellt damit ein spritzbares Knochenersatzmaterial zur Verfügung, das lebende Zellen enthält, bevor es verabreicht wird. Dies unterscheidet den Gegenstand der Erfindung von Knochenersatzmaterialien, die keine Zellen enthalten, sondern erst nach Implantation von Zellen besiedelt werden können. Derartige Materialien, die meist nicht formbar sind und/oder nicht aushärten, können allenfalls osteokonduktiv wirken, d.h. als Leitschiene zur Einsprossung von Knochengewebe. Im Gegensatz dazu wirkt das erfindungsgemäße Knochenersatzmaterial osteogen, d.h. es führt zur Bildung von Knochengewebe aus sich heraus.

Das Knochenersatzmaterial der vorliegenden Erfindung wird in applizierbarer Form, vorzugsweise in spritzbarer Form, zur Verfügung gestellt.

Ein weiterer Aspekt der Erfindung ist ein Verfahren zur Herstellung eines erfindungsgemäßen Knochenersatzmaterials, das die folgenden Merkmale umfaßt:
a) Bereitstellung von lebenden Zellen,
b) Mischen der lebenden Zellen mit einer Zusammensetzung, die Bestandteile zur Bildung einer weichen Matrix enthält,
c) Mischen der lebenden Zellen mit einer Zusammensetzung, die ein aushärtendes Material enthält.

In einer Ausführungsform des Verfahrens werden die lebenden Zellen zunächst mit der Zusammensetzung gemischt, die Bestandteile zur Bildung einer weichen Matrix enthält. Nach Bildung der weichen Matrix werden die darin eingebetteten lebenden Zellen mit der Zusammensetzung gemischt, die ein aushärtendes Material enthält. Der letztgenannte Mischvorgang findet vorzugsweise in einer speziellen Mischkammer statt.

In einer besonderen Ausführungsform umfaßt Schritt c) das Mischen und Applizieren der lebenden Zellen mit einer Zusammensetzung, die ein aushärtendes Material enthält, in einer eigens dafür entwickelten Misch- und Applikationsapparatur in einem Arbeitsgang.

Vorzugsweise handelt es sich bei den Zellen um Vorläuferzellen von Osteoblasten, die durch kleine Knochenbiopsien des Beckens, Brustbeins des Schädels und Kiefers oder von Röhrenknochen gewonnen werden. Aus den Knochenproben wird das lose Stroma herausgespült und nach Zentrifugation in einer Kulturflasche ausplattiert oder in einem Bioreaktor zur Expansion kultiviert. Feste Knochenbestandteile können ebenfalls in Kultur gebracht werden, da hieraus durch Migration weitere Zellen gewonnen werden können. Diese Technik führt zu einer deutlichen Beschleunigung der Zellgewinnung und einer höheren Effizienz der Ausbeute aus der gleichen Materialmenge. Die auswachsenden Zellen werden in der Regel im subkonfluenten Stadium gesplittet und durch zwei- bis dreifache Passagierung vermehrt. Als Alternative zu Knochenproben können auch Aspirate von Knochenmark aus Becken und aus Brustbein verwendet werden. Die Aspirate werden gewöhnlich in Heparinmedium gespült, durch Dichte-Gradientenzentrifugation einer Ficoll- oder Percollsäule von den roten Blutkörperchen getrennt und in einer Kulturflasche ausplattiert.

Vorzugsweise ist eine Lösung, die Bestandteile zur Bildung einer weichen Matrix enthält, eine Fibrinogen enthaltende Lösung. Die weiche Matrix entsteht dann, indem zur Fibrinogenlösung Thrombin zugesetzt wird. Herkömmliche Fibrinkleber, beispielsweise Tissucol®, sind prinzipiell auch anwendbar, jedoch härten diese Fibrinkleber zu einer sehr festen Masse, in der sich Osteoblasten nicht mehr optimal ausdehnen und auch nicht migrieren können; damit kann von den Osteoblasten eine extrazelluläre Matrix nur mehr in verringertem Maß synthetisiert werden.

Bevorzugt ist erfindungsgemäß die weiche Matrix so gestaltet, daß die Zellen sich noch ausdehnen und migrieren können, sowie daß sie auch die Anwesenheit einer erhärtenden Komponente überleben. In einer bevorzugten Ausführungsform des erfindungsgemäßen Verfahrens wird humanes allogenes oder autogenes Fibrinogen in Osteoblasten-Kulturmedium, Phosphatgepufferter Kochsalzlösung (PBS) oder physiologischer Kochsalzlösung (0,9% NaCl) gelöst und gegebenenfalls mit einer weiteren Verbindung, beispielsweise Capronsäure oder anderen, stabilisiert, um eine schnelle enzymatische Fibrinolyse durch Proteinasen zu verhindern. Durch Zugabe von Thrombin in einer Calciumchloridlösung verfestigt und vernetzt sich das Fibrinogen zu Fibrinsträngen. Im Kulturmedium entsteht makroskopisch ein gallertartiges Material, welches in der histologischen Untersuchung mikroskopisch ein dreidimensionales, poröses Netzwerk aus Fibrinsträngen darstellt. Dieses Netzgewebe gewährleistet die Anheftung von Zellen, ihre Migration und dreidimensionale Organisation. Die Senkung der Thrombinkonzentration und die Lösung der Osteoblasten in dem Medium führen zu einer langsameren Formierung des Fibrins, so daß nicht ein homogener solider Clot, sondern vielmehr ein dreidimensionales Fibrinnetzwerk entsteht. Fibrinogen wird bevorzugt in einer Konzentration von 10 bis 150 mg/ml Medium eingesetzt, bevorzugter in einer Konzentration von 10 bis 100 mg/ml Medium, am bevorzugtesten von 50 bis 80 mg/ml Medium. Die bevorzugte Konzentration der Thrombinlösung ist 0,5 bis 1000 I.E./ml Calciumchloridlösung, bevorzugter ist eine Konzentration von 1 bis 40 I.E./ml, die bevorzugteste Konzentration ist 1 bis 10 I.E./ml. Als Stabilisator kann ε-Aminocapronsäure in einer Konzentration von 0,1 bis 10% oder Aprotinin (500 bis 5000 I.E./ml) zur Fibrinogenlösung zugegeben werden.

Vorzugsweise werden die Zellen nach Entfernen des Nährmediums in der oben beschriebenen Fibrinogenlösung suspendiert. Durch Zugabe einer Calciumchlorid-Thrombin-Lösung bildet sich das dreidimensionale Fibrinnetzwerk mit haftenden Osteoblasten im Kulturmedium oder physiologischer Kochsalzlösung. Dabei kann sich der osteoblastische Phänotyp mit dendritischen Zellausläufern bilden, ebenso wie interzelluläre Verbindungen zwischen den Zellen. Mit der Zeit können die Zellen eine extrazelluläre Matrix um sich herum bilden, die später mineralisiert wird. Dabei erhalten die Zellen ihren üblichen Metabolismus und sterben nicht ab.

Die Zellsuspension wird erfindungsgemäß auch mit einer Zusammensetzung gemischt, die ein aushärtendes Material enthält. Bevorzugte aushärtende Materialien sind oben bereits genannt worden. Sie finden auch bei dem erfindungsgemäßen Verfahren Anwendung.

Die verschiedenen Komponenten wie Zellsuspension, Fibrinogen, Thrombin, erhärtende Substanz und weitere können vor dem Vermischen unterschiedlich in Lösungen kombiniert werden. So kann vor dem Mischvorgang Thrombin und Calciumchlorid der Lösung der erhärtenden Substanz beigemengt werden. Fibrinogen ist bevorzugt in der Zellsuspension vorhanden. Die Thrombinlösung kann aber auch eine separate Lösung sein. Die verschiedenen Komponenten können sukzessive miteinander vermischt werden. Vorzugsweise werden sie aber auf einmal miteinander gemischt. In einer besonders bevorzugten Ausführungsform werden die Komponenten unter GMP-Bedingungen in einer Mehrfachspritze bereitgestellt. Dem Anwender steht dann eine zur Injektion/Implantation fertige Apparatur und Masse zur Verfügung, die unter dem Spritzvorgang gemischt wird und damit den Abbindevorgang des Fibrinogens zu Fibrin und des Zementpulvers zu festem Knochenzement einleitet.

Beispielsweise kann in einer Doppelspritze mit einem Mündungsstück eine Spritze mit Calciumphosphatzementpulver in einer Calciumchloridlösung mit Thrombin aufgezogen werden. In der anderen Spritze wird Fibrinogenlösung mit suspendierten Osteoblasten (bevorzugt 1×10⁵ bis 5×10⁶/ml) aufgezogen. In getrenntem Zustand sind die Komponenten ca. 10-15 Minuten haltbar. Durch Spritzen und Zusammenführen der beiden Komponenten in einem gemeinsamen Mündungsstück verbindet sich das Fibrinogen durch Thrombin in Anwesenheit von Calciumionen zu Fibrin. Der Calciumphosphatzement verfestigt sich innerhalb von 15-30 Minuten. Dabei ist zu gewährleisten, daß die Komponenten in einer bestimmten Weise unter Ausbildung von Mikrostrukturen wie beispielsweise interkonnektierenden Poren mit 100 bis 800 µm Porengröße, gemischt werden.

In einer weiteren bevorzugten Ausführungsform wird eine Dreifachspritze verwendet, die folgende Kompartimente enthält:
1. Die zentrale Spritze enthält eine wäßrige Calciumphosphatzementlösung, gegebenenfalls mit folgenden Ergänzungen: Natriumchloridlösung, Milchsäure, Glycerol, Chitosan, Natriumglycerolphosphat, Propylenfumarat, bioaktive Proteine wie Thrombin, Wachstumsfaktoren, Hormone und/oder dafür kodierende Gene in geeigneten Vektoren.
2. Die seitliche Spritze 1 enthält eine Calciumchlorid-Thrombinlösung, gegebenenfalls mit folgenden Zusätzen: Chondroitinsulfat, Proteoglykane, Sialoproteine, Polysaccharide und/oder Wachstumsfaktoren.
3. Die seitliche Spritze 2 enthält eine Fibrinogenlösung und suspendierte Osteoblasten mit gegebenenfalls ε-Aminocapronsäure.

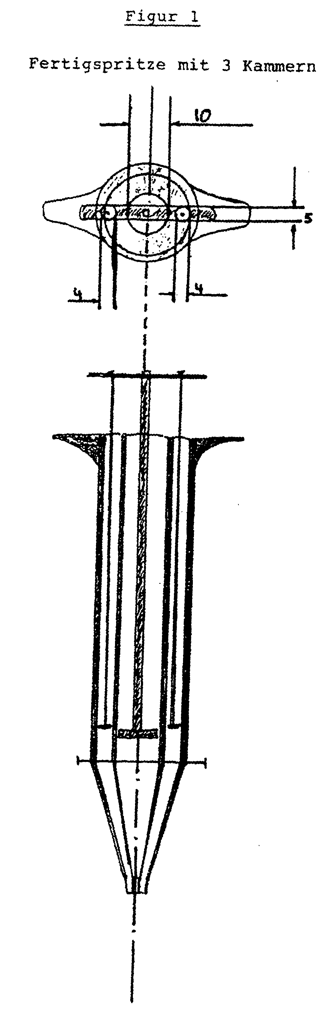

Vorzugsweise kann auch eine Komplettspritze mit drei Kammern zur Anwendung kommen, wie sie in Figur 1 gezeigt ist. Durch synchrones Spritzen wird um einen Calciumphosphatzement-Strahl von 500-2500 µm Durchmesser eine Osteoblasten-Fibrinmatrix gelegt, die in dreidimensionaler Form spongiösem Knochen entspricht.

Dem Fachmann ist klar, daß die Zusammensetzung auch in einer herkömmlichen Einkammer-Spritze appliziert werden kann, nachdem sie vorher aus den verschiedenen Lösungen bzw. Suspensionen durch Mischen hergestellt worden ist. Jedoch ist die separate Mischung der Einzelkomponenten im praktischen Einsatz im OP problematisch, da sich bei eventuellen Verzögerungen in der Anwendung die durchmischten und somit aktivierten Komponenten unweigerlich verfestigen und somit keine optimale Flexibilität in der Anwendung gewährleistet ist.

Ein weiterer Aspekt ist die Verwendung einer Vorrichtung, mit der das spritzbare Knochenmaterial der vorliegenden Erfindung appliziert werden kann. Die Erfindung betrifft daher die Verwendung einer Vorrichtung zur Zubereitung und Verabreichung eines Gemisches umfassend eine Mischkammer mit einer Auslaßöffnung, durch die das Gemisch austreten kann, einen in die Mischkammer führenden ersten Zuleitungskanal (Hauptkanal) und einen oder mehrere in die Mischkammer führende weitere Zuleitungskanäle (Nebenkanäle), wobei das Ende des Nebenkanals bzw. die Enden der Nebenkanäle in der Mischkammer so angeordnet sind, daß aus dem Nebenkanal/den Nebenkanälen in die Mischkammer eintretendes Material in den aus dem Hauptkanal in die Mischkammer eintretenden Materialstrom eindringen kann.

Vorzugsweise hat der Hauptkanal einen Innendurchmesser von mehr als 1 mm, bevorzugter von mehr als 1,5 mm. Durch diesen Zuleitungskanal kann visköses oder hochvisköses Material in die Mischkammer eingebracht werden. Aufgrund des relativ hohen Durchmessers des Hauptkanals können auch lebende Zellen, die in eine visköse Matrix eingebettet sind, in die Mischkammer zugeführt werden, es treten nur sehr niedrige Scherkräfte auf. Vorzugsweise befindet sich die Öffnung des Hauptkanals im Zentrum einer Wand der Mischkammer.

Der Nebenkanal/die Nebenkanäle haben vorzugsweise einen Innendurchmesser von höchstens 1,5 mm und dienen in der Regel der Zuführung niedrigvisköser Materialien in die Mischkammer. Der bevorzugteste Innendurchmesser ist 0,1 bis 1 mm. Die Nebenkanäle können beispielsweise Hohlkanülen mit einem Außendurchmesser von 0,4 bis 1,5 mm sein. Die Anzahl der Nebenkanäle ist wenigstens 1, die bevorzugte Anzahl ist 3 bis 5. Durch die verschiedenen Nebenkanäle kann jeweils das gleiche Material zugeführt werden, es ist aber auch möglich, daß verschiedene Materialien durch die einzelnen Nebenkanäle zugeführt werden. Die Öffnungen des Nebenkanals/der Nebenkanäle in der Mischkammer sind so angeordnet, daß Material, das aus dem Nebenkanal/den Nebenkanälen in die Mischkammer eintritt, in Material, das aus dem Hauptkanal in die Mischkammer eintritt, eindringen kann. Die Öffnungen der Nebenkanäle sind vorzugsweise symmetrisch um die Öffnung des Hauptkanals in der Mischkammer herum angeordnet, so daß aus ihnen austretendes Material in den zentralen Materialstrom, der aus dem Hauptkanal austritt, injiziert wird. Durch diese Injektion findet eine sehr vorteilhafte Durchmischung der niedrigviskösen Komponenten mit der höherviskösen Komponente statt. Dadurch kann beispielsweise direkt während der Anwendung eine reproduzierbare Durchmischung und innige Verbindung der Einzelkomponenten stattfinden. Dabei können Gemische mit interkonnektierenden Strukturen mit einer Porengröße von 100-800 µm erhalten werden. Bislang waren zur Durchmischung von Komponenten stark unterschiedlicher Viskosität wesentlich komplexere Mischvorrichtungen nötig.

Vorzugsweise wird durch den Hauptkanal eine hochvisköse Calciumphosphat-Mischung zugeführt. In einem Nebenkanal kann dann beispielsweise eine Suspension umfassend Fibrinogen und lebende Zellen zugeführt werden. In weiteren Nebenkanälen können zusätzliche Materialien, Zellen wie zum Beispiel Endothelzellen oder weitere Faktoren zugeführt werden.

Ein wichtiger Vorteil der Vorrichtung ist, daß eine Mischung der Komponenten derart erreicht wird, daß eine bevorzugte Mikrostruktur des Materials entsteht, die sich durch Porosität, Interkonnektion der Poren und ein Gerüst, vorzugsweise aus Hydroxylapatit, das ausreichend stabil ist, auszeichnet.

Ein weiterer Vorteil der erfindungsgemäß verwendeten Vorrichtung ist, daß sie so ausgebildet sein kann, daß aufgrund eines sehr geringen Totvolumens nur wenig Material in der Vorrichtung zurückbleibt. Dies kann ein wesentlicher Faktor sein, wenn teure oder schwer ersetzbare Materialien (Zellen) appliziert werden.

In einer besonderen Ausführungsform sind mit den Zuleitungen Vorratsbehältnisse verbunden, aus denen der Inhalt der Vorratsgefäße in die Zuleitungskanäle abgegeben werden kann. Vorzugsweise handelt es sich bei den Vorratsbehältnissen um Spritzen. Das hat den Vorteil, daß medizinisch genormte, sterile Einwegspritzen (Luer-System) verschiedener Größe verwendet werden können. Die Spritzen können in spezielle genormte Anschlüsse gesteckt werden, die an den Enden der Zuleitungskanäle angebracht sind. Die Vorrichtung kann weiter eine Halterung für die Spritzen umfassen sowie eine Stempeleinrichtung, mit der mehrere Stempel der verschiedenen Spritzen gleichzeitig zur Entleerung der Spritzen gedrückt werden können. Die Spritzen, die Halterung und die Stempeleinrichtung können als sterile Einwegteile für medizinische Anwendungen konzipiert werden.

Die Mischkammer, die Zuleitungskanäle sowie die genormten Anschlüsse für die Spritzen sind vorteilhafterweise in einem Bauteil, der Mischeinheit, zusammengefaßt. Sie kann als steriles Einwegteil für medizinische Anwendungen konzipiert werden.

Die Vorrichtung kann Verwendung finden bei der Applikation von injizierbaren biologischen Knochenersatzstoffen, für die die Schaffung von interkonnektierenden Mikrostrukturen minimale Scherkräfte Drücke und Restvolumina die Herstellung unter GMP-Bedingungen sowie einfaches Handling und Herstellung als steriles, preiswertes Einmalbauteil notwendig sind. Die Vorrichtung kann aber auch im Rahmen anderer Konzepte mit ähnlichen Rahmenbedingungen Anwendung finden. Beispiele sind die Applikation von vitalen Zellen in Gelen oder viskösen Systemen. Die Vorteile der beschriebenen Vorrichtung liegen in der Kompatibilität mit gängigen medizinischen Injektionssystemen. Dies erlaubt einerseits die Befüllung von Einwegspritzen mit den verschiedenen Komponenten unter GMP-Bedingungen beim Hersteller und andererseits das Anschließen einer Vielzahl verschiedener Kanülen und Katheter an die Auslaßöffnung der Mischeinheit, so daß eine maximale Flexibilität gewährleistet ist.

Die vorliegende Erfindung stellt eine spritzbare, formbare und sich aushärtende Zusammensetzung pastöser Konsistenz zur Verfügung, die lebende, vorzugsweise autogene Zellen zur Knochenbildung enthält, die zum Schutz in einer biologischen Gelmatrix eingehüllt sind. Diese weiche Matrixkomponente gewährleistet zudem eine plastische Formbarkeit der Paste in konkrete dreidimensionale Formen und in vivo das Ausbreiten von synthetisierter Knochensubstanz als auch das Einsprossen von Blutgefäßen. Die aushärtende Matrix gewährleistet die Formstabilität und Druckfestigkeit des Konstrukts. Die bevorzugte Zusammensetzung aus Calciumphosphaten stimuliert die Osteoblasten zur Ausreifung und Synthese von extrazellulärer Knochenmatrix, für die sie die Calcium- und Phosphationen bereitstellt. In vivo führt das erfindungsgemäße Material zur Knochenbildung aus sich heraus.

Figur 1 zeigt eine Dreikammerspritze, durch die das erfindungsgemäße Knochenersatzmaterial gemischt und appliziert werden kann.

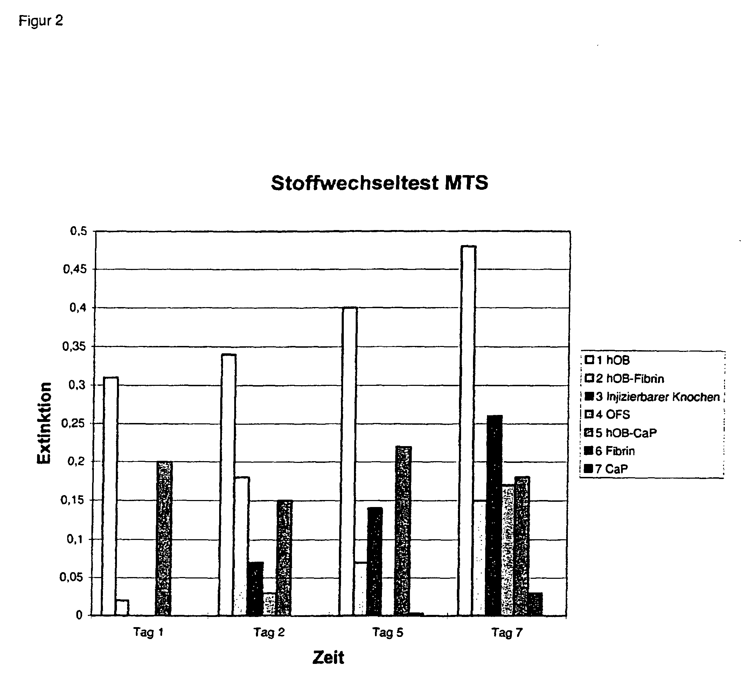

Figur 2 zeigt eine graphische Darstellung der Ergebnisse eines MTS-Stoffwechseltests. Die Experimente sind in Beispiel 6 beschrieben. Das Ergebnis belegt, daß die Zellen in dem erfindungsgemäßen Knochenersatzmaterial über einen beträchtlichen Zeitraum stoffwechselaktiv sind und überleben.

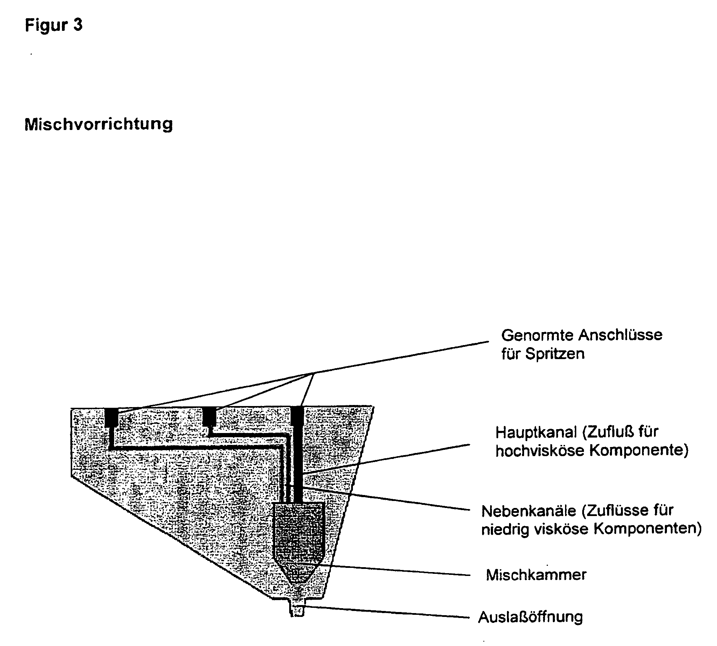

Figur 3 zeigt eine schematische Darstellung einer Mischeinheit, in der die einzelnen Bauelemente (Haupt- und Nebenkanäle, Anschlüsse für Spritzen, Mischkammer mit Auslaßöffnung) in einem Bauteil zusammengefaßt sind. Durch den Hauptkanal kann als hochvisköse Komponente beispielsweise eine Calciumphosphat-Knochenzement-Mischung zugeführt werden. Durch die Nebenkanäle können niedrig-visköse Komponenten zugeführt werden, z.B. Osteoblasten-Fibrinogen-Suspension, zusätzliche Wachstums- und Differenzierungsfaktoren, Plasmide und andere Komponenten.

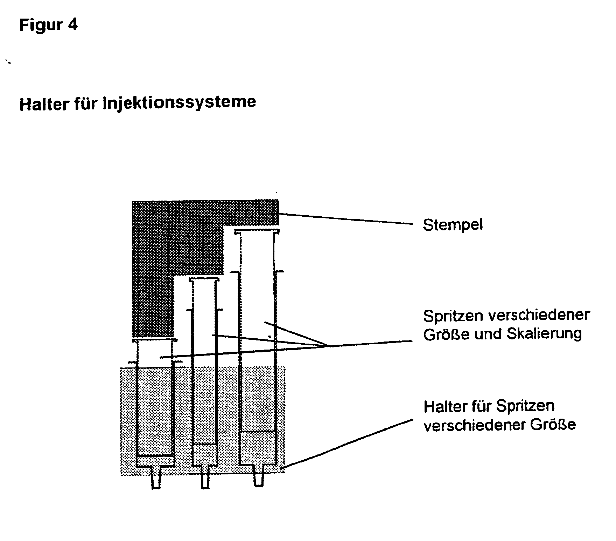

Figur 4 zeigt eine schematische Darstellung eines Bauteils umfassend mehrere Spritzen, die von einem Halter in einer bestimmten Anordnung gehalten werden, und einen Stempel, mit dem die einzelnen Stempel der Spritzen synchron bewegt werden können. Ein derartiges Bauteil, auch Applikator genannt, kann an eine Mischeinheit (Figur 3) angeschlossen werden.

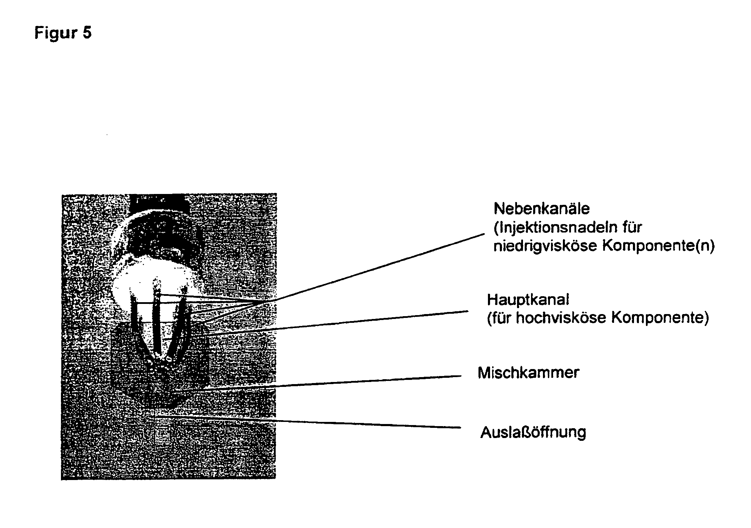

Figur 5 zeigt die Darstellung einer bevorzugten Ausführungsform der erfindungsgemäßen Vorrichtung. Die Öffnung des Hauptkanals ist symmetrisch von den in die Mischkammer ragenden Enden der 6 Nebenkanäle umgeben, die leicht nach innen gebogen sind, so daß aus den Nebenkanälen austretendes Material in den aus dem Hauptkanal austretenden Materialstrom injiziert wird. Durch den Hauptkanal kann als hochvisköse Komponente beispielsweise eine Calciumphosphat-Knochenzement-Mischung zugeführt werden. Durch die Nebenkanäle können niedrig-visköse Komponenten zugeführt werden, z.B. Osteoblasten-Fibrinogen-Suspension, zusätzliche Wachstums- und Differenzierungsfaktoren, Plasmide und andere Komponenten.

Die folgenden Beispiele sollen die Erfindung näher erläutern.

### Beispiel 1

### 1. Etablierung einer Osteoblastenkultur

Es stehen verschiedene Methoden zur Verfügung, erstens die offene Knochenbiopsie (als Migrations- oder stromale Zellkultur) und zweitens die Aspiration von Knochenmark, die im folgenden dargestellt werden.

### a) Knochenmarkbiopsie

Es werden sterile Knochenbiopsien in örtlicher Betäubung durch einen Hohlbohrer entnommen. Nach einem kleinen Hautschnitt werden Spongiosablöckchen von 0,5 bis 1 cm³ entnommen, die Wunde wird verschlossen. Eine weitere Möglichkeit ist die Aspiration von ca. 15 ml Knochenmark.

Die Spongiosa sollte sehr schnell weiterverarbeitet werden und wenn möglich nicht länger als 12 Stunden im Transportgefäß bei 4°C lagern. Das Medium wird verworfen, die Spongiosa in die Petrischale gegeben und dort in 2 bis 3 mm kleine Partikel (Chips) zerkleinert.

### aa) Migrationskultur

Es werden ca. 3 bis 4 Partikel in eine Vertiefung einer 6-Well-Platte verteilt und mit 3 ml Medium aufgefüllt, bzw. 6 bis 7 Chips pro 25 cm² mit 7 ml Medium. Die Inkubation erfolgt im Brutschrank bei 37°C und 5% CO₂. Der Mediumwechsel sollte zweimal pro Woche erfolgen, wobei eine Inspektion unter dem Phasenkontrastmikroskop durchgeführt wird. Nach 5 bis 9 Tagen sind erste Zellen zu erkennen, nach 10 bis 14 Tagen ein subkonfluenter Zellayer mit 65 bis 75% Bodenflächenbedeckung.

### bb) Stromale Zellkultur (eigene Modifikation)

Zunächst wird der Knochen von Muskel-/Bindegewebsresten befreit. Die Spongiosa wird mit Schere und Pinzette in möglichst kleine Stückchen zerkleinert. Die Spongiosafragmente können (ohne Medium) in ein 50 ml-Falcon-Gefäß (ein Polypropylen-Schraubgefäß) gegeben und damit gewogen werden. Enthält das Material viel rotes Knochenmark, so kann man später pro 4 bis 6 g Spongiosa eine 75 cm²-Kulturflasche bestücken. In das 50 ml-Falcon-Gefäß wird nun zur zerkleinerten Spongiosa ca. 25 ml Medium gegeben und durch Vortexen (hochfrequenter Rüttelvorgang, ca. 30 Sekunden, höchste Stufe) die Zellen herausgelöst. Der Überstand wird in andere 50 ml-Falcon-Gefäße überführt. Dieser Schritt wird wiederholt, bis das Medium nach Schütteln (Vortexen) nicht mehr trüb wird. Zuletzt kann noch eine Trypsin(-Collagenase)-Behandlung (ca. 10 Minuten, 37°C) durchgeführt werden, um weitere Zellen zu gewinnen. Die erhaltenen Zellsuspensionen werden bei 250 g für 10 Minuten bei 4°C zentrifugiert. Überstände werden verworfen und die Zellpellets in Medium resuspendiert und auf Kulturflaschen verteilt. Die gespülten Knochenstückchen können gegebenenfalls in einer separaten Kulturflasche zur Anzüchtung restlicher Zellen verwendet werden (nach Trypsinierung sollten diese aber gut mit Medium gespült werden).

Verbesserte Zellverteilung durch Auszählung der kernhaltigen Zellen:
Von der nach Resuspension der Zellpellets erhaltenen Zellsuspension werden 50 µl in ein Eppendorf-Gefäß überführt und mit 46 µl Trypanblau gemischt. Anschließend folgt die Zugabe von 4 µl Essigsäure zur Lyse der Erythrozyten (Endkonzentration 4% Essigsäure). Die kernhaltigen Zellen werden in einer Neubauer-Zählkammer ausgezählt.
Beispielsweise 3×10⁵ bis 4×10⁵ Zellen der Zellsuspension werden pro 75 cm²-Kulturflasche verteilt.
Mediumwechsel findet erst nach 4 bis 5 Tagen statt, danach zweimal pro Woche.
Am zweiten Tag sind erste adhärente Zellen vorhanden, am vierten bis fünften Tag Klone. Die Subkonfluenz tritt meist zwischen dem neunten und dem elften Tag ein.

### b) Isolierung und Selektion der Knochenvorläuferzellen aus Knochenmarkaspirat

Bei örtlicher Betäubung wurde eine sterile Knochenmarkpunktion durchgeführt. Die Aspiration von 15 bis 20 ml Blut aus dem hinteren Beckenkamm wurde mit einer großvolumigen Kanüle und einer Heparin-benetzten Spritze durchgeführt. Danach wurde bei 1200 U/min zentrifugiert, der Überstand entnommen und das Zellpellet in 5 ml serumfreiem Medium resuspendiert. Die entstandene Suspension wurde über einen Dichte-Gradienten (70% Percollsäule, Ficollgradient) einer Dichte-Zentrifugation unterzogen, die Mesenchymzellen wurden im Kulturgefäß ausplattiert und die Zellen durch Subkultivierung vermehrt.

### c) Nachweis des osteoblastischen Phänotyps

Der osteoblastische Phänotyp wird durch die knochenspezifischen Proteine Alkalische Phosphatase und Osteocalcin im Kulturmedium (Ablauf) und durch immunhistochemische Färbungen von Kontrollkulturen nachgewiesen.

### Beispiel 2

### Herstellung eines Fibrinklebers

66 mg Fibrinogen werden in 1 ml Kulturmedium (αMEM oder Medium 199 oder BGJ-B-Medium) ohne Serumzusatz mit 100 U/ml Penicillin und 100 mg/ml Streptomycin oder physiologischer Kochsalzlösung gelöst. ε-Amino-n-Capronsäure wird in einer Endkonzentration von 0,1 bis 10% der allogenen oder autogenen Fibrinogenlösung zugesetzt. 1,25 I.E. Thrombin werden in 40 µl Calciumchlorid-Lösung (40 mM) gelöst. Schließlich wird 1 ml der Fibrinogen-Lösung mit 60 µl Calciumchlorid-Thrombin-Lösung gemischt. Die Mischung wird dann in eine Kulturschale eingespritzt.

### Beispiel 3

### Herstellung einer Osteoblasten-Fibrinsuspension

Humane Osteoblasten und deren Vorläuferzellen werden aus einer Knochenmarkbiopsie gewonnen und ex vivo vermehrt, wie in Beispiel 1 beschrieben. Die subkonfluente Zellkultur in einer 75 cm²-Kulturflasche wird mit 1 ml 0,025% Trypsin/EDTA-Lösung für 5 Minuten trypsinisiert. Die Zellsuspension wird in 2 ml Medium mit 10% FCS aufgenommen und 5 Minuten bei 1000 U/min und 4°C zentrifugiert. Das Zellpellet wird in 100 µl Medium resuspendiert. Nach Bestimmung der Zellzahl werden 20.000 Osteoblasten (Zellpassage 1 bis 3) in 200 µl allogener oder autogener Fibrinogenlösung aus Beispiel 2 suspendiert.

Anschließend wird 60 µl der Calciumchlorid-Thrombin-Lösung aus Beispiel 2 zugegeben. Das Gemisch wird in eine Kulturschale oder in die Vertiefungen einer 48-Well-Platte gespritzt. Nach Zugabe von 760 µl Kulturmedium BGJ-B mit 10% FCS und 100 U/ml Penicillin und 100 mg/ml Streptomycin werden die Zellen im Wärmeschrank bei 37°C, 5% CO₂ und 100% Luftfeuchtigkeit kultiviert.

Im Lichtmikroskop zeigt sich nach 24 bis 72 Stunden bei 100-facher Vergrößerung die Ausbildung des osteoblastischen Phänotyps mit dendritischen Zellausläufern und nach 5 bis 12 Tagen der Aufbau von interzellulären Verbindungen der Zellen. Im Verlauf bilden die Zellen eine extrazelluläre Matrix um sich, die später mineralisiert wird. Die Vitalität der Zellen kann durch Trypanblaufärbung überprüft werden. Dazu wird der Überstand an Kulturmedium abgesaugt, anschließend werden 50 µl Trypanblaulösung zugegeben. Die Zellen werden dann unter dem Lichtmikroskop untersucht. Bei Trypanblaufärbung finden sich auch nach Wochen nur wenige abgestorbene Zellen.

### Beispiel 4

### Herstellung einer Hydroxylapatit-Osteoblastenmischung

Osteoblasten wurden in Medium suspendiert und auf nichtkeramisches Hydroxylapatit gegeben, welches aus Calciumphosphat hergestellt wurde. In der Kultur hafteten die Zellen an den Hydroxylapatit-Partikeln. Im Elektronenmikroskop zeigte sich eine Adhäsion der Zellen auf der kristallinen Oberfläche. Im Stoffwechseltest zeigte sich ein erhaltener Zellmetabolismus der anhaftenden Zellen.

### Beispiel 5

### Herstellung einer Hydroxylapatit-Zement-Fibrin-Matrix

Als fester Bestandteil wurde der Fibrin-Suspension ein Zement aus Calciumphosphat zugegeben. Dazu wurde zunächst untersucht, ob der in Wasser gelöste Zement sich mit dem Fibrin/Thrombin/Calciumchlorid-Komplex vermischen und als Paste spritzen läßt. Es gelang, die Mischung zu spritzen und sie anschließend zu formen (Primäre Stabilität). Sie behielt die gegebene Form und verfestigte sich in Minuten zu einer festen Substanz (Sekundäre Stabilität).

### Beispiel 6

### Herstellung einer Osteoblasten-Fibrin-Calciumphosphat-Zement-Paste

### a) Fibrinogenlösung:

66 mg Fibrinogen werden in 1 ml Kulturmedium (αMEM oder Medium 199 oder BGJ-B-Medium) ohne Serumzusatz mit 100 U/ml Penicillin und 100 mg/ml Streptomycin oder physiologischer Kochsalzlösung gelöst. ε-Amino-n-capronsäure wird in einer Endkonzentration von 0,1 bis 10% der Fibrinogenlösung zugesetzt.

### b) Fibrinogen-Osteoblastensuspension:

Die Zellkultur wird etabliert, wie in Beispiel 1 beschrieben. Die subkonfluente Zellkultur wird trypsinisiert, in Medium suspendiert und zentrifugiert (siehe Beispiel 3). Das Zellpellet wird in 100 µl Medium resuspendiert. Nach Zellzählung werden 20.000 Osteoblasten (Zellpassage 1 bis 3) in 200 µl Fibrinogenlösung suspendiert.

### c) Calciumphosphat-Thrombin-Calciumchloridlösung:

1,25 I.E. Thrombin werden in 0,5 ml 40 mM Calciumchloridlösung gelöst. Anschließend wird 1 g Calciumphosphat-Pulver (BoneSource® ) zu 0,5 ml der Calciumchlorid-Thrombin-Lösung zugegeben.

### d) Mischung der Komponenten:

500 µl Fibrinogen-Osteoblasten-Suspension und 500 µl Calciumghosphat-Thrombin-Calciumchlorid-Lösung werden in eine 1 ml Spritze eingebracht. Die Spritze wird dann etwa 10 Sekunden geschüttelt, wonach jeweils etwa 200 µl in eine Kulturschale oder 48-Well-Platte gespritzt werden. Danach wird 800 µl Kulturmedium BGJ-B mit 10% FCS und 100 U/ml Penicillin und 100 mg/ml Streptomycin zugegeben. Die Zellen werden, wie oben beschrieben, im Wärmeschrank inkubiert.

### e) Stoffwechseltest MTS (Cell Proliferation Assay):

Der Cell Proliferation Assay der Firma Boehringer Mannheim wurde benutzt. Er beruht auf der Transformation eines Tetrazoliumsalzes MTS zu einem gelbgefärbten Formazan durch die mitochondriale Dehydrogenase. Es handelt sich um einen kalorimetrischen Stoffwechseltest. Die Ansätze beinhalteten 20.000 menschliche Osteoblasten (hOB) pro Ansatz (48-Well), 200 µl Fibrinogenlösung (66 mg/ml) und 200 µg Calciumphosphatpulver (CaP) in 200 µl Calciumchlorid-Thrombin-Lösung. Nach Absaugen des Kulturmediums wurde 1 ml MTS-Lösung zugegeben. Der Farbumschlag in jeweils 100 µl MTS-Lösung wurde nach drei Stunden photometrisch bestimmt. Die verschiedenen Ansätze und Kontrollen sind in der folgenden Tabelle I wiedergegeben:

| Ansatz | hOB | hOB-Fibrin | Injizierbarer Knochen | hOB-Fibrin OFS | hOB-CaP | Fibrin | CaP |
|---|---|---|---|---|---|---|---|
| | | getrennt | gemischt | gemischt | gemischt | | |
| | 1 | 2 | 3 | 4 | 5 | 6 | 7 |
| 20.000 hOB | x | x | x | x | x | | |
| 200 µl Fibrinogen | | x | x | x | | x | |
| 200 µg CaP | | | x | | x | | x |

Ansätze 2 und 4 enthalten die gleichen Komponenten hOB und Fibrin, in Ansatz 2 ungemischt ("getrennt"), in Ansatz 4 als Osteoblasten-Fibrin-Suspension OFS gemischt. Die getrennte Prüfung der Komponenten in einem Ansatz soll zum einen ermöglichen einen negativen oder positiven Effekt auf die Zellen gegenüber der unbehandelten Zellkontrolle (Ansatz 1) oder eine Absorption des Farbstoffs durch das Fibrin zu entdecken. Ist die Zellzahl im Ansatz 2, die parallel kontrolliert wird, gleich zum Ansatz 1 und die Absorption im MTS-Test sinkt, so absorbiert Fibrin den Farbstoff. Sinkt die Zellzahl durch reine Zugabe des Fibrins in den Ansatz mit Zellen, so ist ein toxischer Effekt auf die hOB anzunehmen. Im vorliegenden Fall war die Zellzahl konstant, und Fibrin absorbiert den zu messenden Farbstoff teilweise, ein toxischer Effekt wurde ausgeschlossen. Das heißt auch, daß der geringere Wert für OFS (Ansatz 4) gegenüber der Zellkontrolle (Ansatz 1) nicht nur durch Einbringen der Zellen in das Fibrin, sondern auch durch Absorption zu erklären ist. Die reale Stoffwechselaktivität liegt damit höher als der Extinktionswert angibt.

Die Ergebnisse des Stoffwechseltests sind in folgender Tabelle II gezeigt:

| Ansatz | Tag 1 | Tag 2 | Tag 5 | Tag 7 |
|---|---|---|---|---|
| 1 hOB | 0,31 | 0,34 | 0,4 | 0,48 |
| 2 hOB-Fibrin | 0,02 | 0,18 | 0,07 | 0,15 |
| 3 Injizierbarer Knochen | 0 | 0,07 | 0,14 | 0,26 |
| 4 OFS | 0 | 0,03 | 0 | 0,17 |
| 5 hOB-CaP | 0,2 | 0,15 | 0,22 | 0,18 |
| 6 Fibrin | 0 | 0 | 0,003 | 0,03 |
| 7 CaP | 0 | 0 | 0 | 0 |

Figur 2 zeigt eine graphische Darstellung der Ergebnisse.

### Beispiel 7

### In vivo-Versuch

Die Gewinnung und Vermehrung der Osteoblasten erfolgt wie in Beispiel 1 beschrieben. Die Zellen werden durch Trypsin-Lösung enzymatisch abgelöst. Die Osteoblasten (1 × 10⁶/ml) werden in einer Fibrinogen-Lösung (66 mg/ml) suspendiert. 500 mg Calciumphosphatzement BoneSource® werden in 0,5 ml Calciumchlorid-Lösung (40 mM) mit 1,25 I.E. Thrombin/ml gelöst. 0,5 ml Fibrinogen-Osteoblasten-Suspension werden mit 0,5 ml Calciumphosphat-Thrombin-Calciumchlorid-Lösung in eine 1 ml-Spritze gefüllt und anschließend subcutan in eine Nacktmaus injiziert.

Ca. 6 bis 8 Wochen alte Nacktmäuse wurden in einer Narkosekammer mit einem Isofluran®-Sauerstoff-Gemisch (3% Isofluran in 100% O₂, Flow 4 1/min) betäubt. Unter Aufrechterhaltung der Narkose mit einer Inhalationsmaske (1,5 bis 2 Vol.-% Isofluran in 100% O₂, Flow 0,5 bis 1 1/min) wurden die Tiere mit Betaisodona® abgewaschen, das Operationsgebiet rasiert und steril abgedeckt. Es erfolgte ein ca. 4 mm langer quer verlaufender Hautschnitt im Bereich des Rückens. Der Schnitt wurde mit einer Präparationsschere gespreizt, und es wurde eine Hauttasche geschaffen. Ein Spritzkonus wurde eingeführt und die Paste unter die Haut der Tiere injiziert. Die Paste wurde durch perkutane manuelle Formung zu Längssträngen geformt. Die Wunde wurde mit Einzelknopfnähten verschlossen und ein steriler Wundverband angelegt. Die Dauer des Eingriffs betrug ca. 15 Minuten. Die Wunden der Tiere wurden bis zur gesicherten Wundheilung täglich kontrolliert.

Die Nacktmäuse erhielten schließlich eine letale Dosis CO₂ per inhalationem am 14., 29. und 48. Tag postoperativ. Die Konstrukte wurden mit dem umgebenden Gewebe herauspräpariert, photographiert und danach histologisch und immunhistochemisch aufgearbeitet. Die Ergebnisse sind in der nachfolgenden Tabelle III zusammengefaßt:

| **Maus Nr**. | **Tag** | **Form** | **Festigkeit** | **Matrixsynthese** | **Vaskular**. |
|---|---|---|---|---|---|
| 1 | 14 | konstant | druckstabil | Bindegewebe | ja |
| 2 | 29 | konstant | druckstabil | Beg. Knochen | ja |
| 3 | 48 | konstant | druckstabil | Knochengewebe | ja |
| 4 | 29 | konstant | druckstabil | Beg. Knochen | ja |

"Vaskular." steht für Vaskularisierung, "Beg. Knochen" steht für beginnende Knochenbildung und beschreibt die Änderung der Morphologie von undifferenziertem Bindegewebe zu differenziertem Knochengewebe.

Die Konstrukte waren größenkonstant und in der Form unverändert nach 14, 29 und 48 Tagen.

Histologisch fand sich am
- 14. Tag eine Gefäßeinsprossung und extrazelluläre Bindegewebsmatrix;
- 29. Tag ein Gefäßnetz und beginnende Knochenbildung im Konstrukt;
- 48. Tag ein Gefäßnetz und Knochengewebe.

Grundsätzlich sind die angegebenen Konzentrationen variierbar, um unterschiedliche Qualitäten bezüglich Zelldichte, Festigkeit und Formbarkeit zu erreichen. Eine Anpassung an die lokalen Bedingungen in einem Knochendefekt wie Druckbelastung, Scherkräfte, Volumen ist möglich.

## Patentansprüche

1. Knochenersatzmaterial, umfassend wenigstens folgende Komponenten:
a) eine weiche Matrix, die Fibrin oder Fibrinogen enthält,
b) lebende Zellen,
c) eine aushärtende Matrix, die nicht-keramischen Hydroxylapatit-Zement enthält.

2. Knochenersatzmaterial nach Anspruch 1, **dadurch gekennzeichnet, daß** die weiche Matrix Thrombin enthält.

3. Knochenersatzmaterial nach Anspruch 2, **dadurch gekennzeichnet, daß** die weiche Matrix ε-Aminocapronsäure oder Aprotinin enthält.

4. Knochenersatzmaterial nach einem der vorhergehenden Ansprüche, **dadurch gekennzeichnet, daß** die weiche Matrix wenigstens eine Substanz enthält, die ausgewählt ist aus der Gruppe umfassend Chondroitinsulfat, Proteoglykane, Sialoproteine, Wachstumsfaktoren, Hormone und Nucleinsäuren kodierend für Wachstumsfaktoren oder Hormone.

5. Knochenersatzmaterial nach einem der vorhergehenden Ansprüche, **dadurch gekennzeichnet, daß** die weiche Matrix wenigstens eine Substanz enthält, die ausgewählt ist aus der Gruppe umfassend biologische Kollagengele, Gelatine, Alginate, Agarose, Polysaccharide, synthetisches Kollagen, Hydrogele und visköse Polymere.

6. Knochenersatzmaterial nach einem der vorhergehenden Ansprüche, **dadurch gekennzeichnet, daß** wenigstens ein wesentlicher Teil der lebenden Zellen Osteoblasten oder deren Vorläuferzellen sind.

7. Knochenersatzmaterial nach einem der vorhergehenden Ansprüche, **dadurch gekennzeichnet, daß** es weiterhin lebende gefäßbildende Zellen enthält.

8. Knochenersatzmaterial nach Anspruch 7, **dadurch gekennzeichnet, daß** die gefäßbildenden Zellen Endothelzellen oder deren Vorläuferzellen sind.

9. Knochenersatzmaterial nach einem der vorhergehenden Ansprüche, **dadurch gekennzeichnet, daß** die aushärtende Matrix wenigstens eine Substanz enthält, die sich durch Kristallisation zu Hydroxylapatit verbindet.

10. Knochenersatzmaterial nach einem der Ansprüche 1 bis 9, **dadurch gekennzeichnet, daß** die aushärtende Matrix PGLA enthält.

11. Knochenersatzmaterial nach einem der vorhergehenden Ansprüche, **dadurch gekennzeichnet, daß** sich die aushärtende Matrix innerhalb von 15 Minuten verfestigt.

12. Knochenersatzmaterial nach einem der vorhergehenden Ansprüche, **dadurch gekennzeichnet, daß** es
in einer Mehrfachspritze bestehend aus mehreren Spritzen, die kombiniert sind, oder
in einer Komplettspritze mit mehreren Kammern
bereitgestellt wird.

13. Verfahren zur Herstellung eines Knochenersatzmaterials umfassend eine weiche Matrix, lebende Zellen und eine aushärtende Matrix gemäß einem der Ansprüche 1 bis 12, **dadurch gekennzeichnet, daß** es die folgenden Merkmale aufweist:
a) Bereitstellung von lebenden Zellen,
b) Mischen der lebenden Zellen mit einer Zusammensetzung, die Bestandteile zur Bildung einer weichen Matrix enthält, und
c) Mischen der lebenden Zellen mit einer Zusammensetzung, die ein aushärtendes Material enthält.

14. Verfahren nach Anspruch 13, **dadurch gekennzeichnet, daß** zunächst die lebenden Zellen mit der Zusammensetzung, die Bestandteile zur Bildung einer weichen Matrix enthält, gemischt werden, und anschließend die in der weichen Matrix eingebetteten lebenden Zellen mit der Zusammensetzung, die ein aushärtendes Material enthält, gemischt werden.

15. Verfahren nach Anspruch 13 oder 14, **dadurch gekennzeichnet, daß** die lebenden Zellen durch eine Knochenbiopsie oder Knochenmarksaspiration gewonnen werden.

16. Verfahren nach einem der Ansprüche 13 bis 15, **dadurch gekennzeichnet, daß** die lebenden Zellen vor Schritt b) und c) in vitro kultiviert werden.

17. Verfahren nach einem der Ansprüche 13 bis 16, **dadurch gekennzeichnet, daß** wenigstens ein Teil der lebenden Zellen Osteoblasten oder deren Vorläuferzellen sind.

18. Verfahren nach einem der Ansprüche 13 bis 17, **dadurch gekennzeichnet, daß** die weiche Matrix durch Inkontaktbringen einer Fibrinogenlösung und einer Thrombinlösung hergestellt wird.

19. Verfahren nach Anspruch 18, **dadurch gekennzeichnet, daß** das Fibrinogen zunächst in Osteoblastenmedium oder physiologischer Kochsalzlösung (0,9% NaCl) oder Phosphatgepufferter Kochsalzlösung (PBS) gelöst wird.

20. Verfahren nach Anspruch 18 oder 19, **dadurch gekennzeichnet, daß** die Fibrinogenlösung durch ε-Aminocapronsäure stabilisiert wird.

21. Verfahren nach einem der Ansprüche 13 bis 20, **dadurch gekennzeichnet, daß** zu einer der Zusammensetzungen, mit denen die lebenden Zellen gemischt werden, wenigstens eine Substanz zugegeben wird, die ausgewählt ist aus der Gruppe umfassend Chondroitinsulfat, Proteoglykane, Sialoproteine, Wachstumsfaktoren, Hormone und Nucleinsäuren kodierend für Wachstumsfaktoren oder Hormone.

22. Verfahren nach einem der Ansprüche 13 bis 21, **dadurch gekennzeichnet, daß** zu einer der Zusammensetzungen, mit denen die lebenden Zellen gemischt werden, wenigstens eine Substanz zugesetzt wird, die ausgewählt ist aus der Gruppe umfassend biologische Kollagengele, Gelatine, Alginate, Agarose, Polysaccharide, synthetisches Kollagen, Hydrogele und visköse Polymere.

23. Verfahren nach einem der Ansprüche 13 bis 22, **dadurch gekennzeichnet, daß** die aushärtende Matrix durch Lösen von nicht-keramischem Hydroxylapatit-Zement in einer wäßrigen Lösung hergestellt wird.

24. Verfahren nach einem der Ansprüche 13 bis 23, **dadurch gekennzeichnet, daß** das Knochenersatzmaterial
in einen Mehrkomponentenapplikator, bestehend aus mehreren Containern, die auch Spritzen sein können, die kombiniert sind, oder
in eine Komplettspritze mit mehreren Kammern
gegeben wird.

25. Verfahren nach einem der Ansprüche 13 bis 23, **dadurch gekennzeichnet, daß** das Knochenersatzmaterial in einer Vorrichtung zur Zubereitung und Verabreichung des Knochenersatzmaterials hergestellt wird, wobei die Vorrichtung folgende Merkmale umfasst
a) eine Mischkammer mit einer Auslaßöffnung, durch die das Gemisch austreten kann,
b) einen in die Mischkammer führenden ersten Zuleitungskanal (Hauptkanal) und
c) einen oder mehrere in die Mischkammer führende weitere Zuleitungskanäle (Nebenkanäle),
wobei das Ende des Nebenkanals/die Enden der Nebenkanäle in der Mischkammer so angeordnet sind, daß aus dem Nebenkanal/den Nebenkanälen in die Mischkammer eintretendes Material in den aus dem Hauptkanal in die Mischkammer eintretenden Materialstrom eindringen kann.

26. Verfahren nach Anspruch 25, dadurch gezeichnet, daß bei der Vorrichtung der Inndendurchmesser des Hauptkanals wenigstens 1 mm beträgt.

27. Verfahren nach Anspruch 25 oder 26, **dadurch gekennzeichnet, daß** bei der Vorrichtung der Innendurchmesser des Nebenkanalslder Nebenkanäle höchstens 1,5 mm beträgt.

28. Verfahren nach einem der Anspruche 25 bis 27, **dadurch gekennzeichnet, daß** die Vorrichtung 3 bis 5 Nebenkanäle umfaßt.

29. Verfahren nach einem der Ansprüche 25 bis 28, **dadurch gekennzeichnet, daß** bei der Vorrichtung die Enden der Nebenkanäle in der Mischkammer im wesentlichen symmetrisch um das Ende des Hauptkanals in der Mischkammer herum angeordnet sind.

30. Verfahren nach einem der Ansprüche 25 bis 29, **dadurch gekennzeichnet, daß** bei der Vorrichtung sich das Ende des Nebenkanals/die Enden der Nebenkanäle in der Mischkammer mit der gedachten Verlängerung des Hauptkanals in der Mischkammer überschneiden.

31. Verfahren nach einem der Ansprüche 25 bis 30, **dadurch gekennzeichnet, daß** bei der Vorrichtung mit den Zuleitungskanälen Vorratsbehältnisse verbunden sind, aus denen der Inhalt der Vorratsbehältnisse in die Zuleitungskanäle abgegeben werden kann.

32. Verfahren nach Anspruch 31, **dadurch gekennzeichnet, daß** bei der Vorrichtung die Vorratsbehältnisse Spritzen sind.

## Claims

1. A bone substitute material comprising at least the following components:
a) a soft matrix comprising fibrin or fibrinogen,
b) living cells,
c) a setting matrix comprising non-ceramic hydroxyapatite cement.

2. A bone substitute material as claimed in claim 1, wherein the soft matrix comprises thrombin.

3. A bone substitute material as claimed in claim 2, wherein the soft matrix comprises ε-aminocaproic acid or aprotinin.

4. A bone substitute material as claimed in any of the preceding claims, wherein the soft matrix comprises at least one substance which is selected from the group comprising chondroitin sulfate, proteoglycans, sialoproteins, growth factors, hormones and nucleic acids coding for growth factors or hormones.

5. A bone substitute material as claimed in any of the preceding claims, wherein the soft matrix comprises at least one substance selected from the group comprising biological collagen gels, gelatin, alginates, agarose, polysaccharides, synthetic collagen, hydrogels and viscous polymers.

6. A bone substitute material as claimed in any of the preceding claims, wherein at least one essential part of the living cells are osteoblasts or their precursor cells.

7. A bone substitute material as claimed in any of the preceding claims, which additionally comprises living angiogenic cells.

8. A bone substitute material as claimed in claim 7, wherein the angiogenic cells are endothelial cells or their precursor cells.

9. A bone substitute material as claimed in any of the preceding claims, wherein the setting matrix comprises at least one substance which binds together by crystallization to give hydroxyapatite.

10. A bone substitute material as claimed in any of claims 1 to 9, wherein the setting matrix comprises PGLA.

11. A bone substitute material as claimed in any of the preceding claims, wherein the setting matrix solidifies within 15 minutes.

12. A bone substitute material as claimed in any of the preceding claims, wherein it is provided
in a multiple syringe consisting of a plurality of syringes which are combined, or
in a complete syringe with a plurality of chambers.

13. A process for producing a bone substitute material comprising a soft matrix, living cells and a setting matrix as claimed in any of claims 1 to 12, which comprises the following features:
a) preparation of living cells,
b) mixing of the living cells with a composition which comprises constituents to form a soft matrix, and
c) mixing of the living cells with a composition which comprises a setting material.

14. A process as claimed in claim 13, wherein initially the living cells are mixed with the composition which comprises constituents to form a soft matrix, and then the living cells embedded in the soft matrix are mixed with the composition which comprises a setting material.

15. A process as claimed in claim 13 or 14, wherein the living cells are obtained by a bone biopsy or bone marrow aspiration.

16. A process as claimed in any of claims 13 to 15, wherein the living cells are cultivated in vitro before step b) and c).

17. A process as claimed in any of claims 13 to 16, wherein at least part of the living cells are osteoblasts or their precursor cells.

18. A process as claimed in any of claims 13 to 17, wherein the soft matrix is produced by bringing a fibrinogen solution and a thrombin solution into contact.

19. A process as claimed in claim 18, wherein the fibrinogen is initially dissolved in osteoblast medium or physiological saline (0.9% NaCl) or phosphate-buffered saline (PBS).

20. A process as claimed in claim 18 or 19, wherein the fibrinogen solution is stabilized by ε-aminocaproic acid.

21. A process as claimed in any of claims 13 to 20, wherein at least one substance selected from the group comprising chondroitin sulfate, proteoglycans, sialoproteins, growth factors, hormones and nucleic acids coding for growth factors or hormones is added to one of the compositions with which the living cells are mixed.

22. A process as claimed in any of claims 13 to 21, wherein at least one substance selected from the group comprising biological collagen gels, gelatin, alginates, agarose, polysaccharides, synthetic collagen, hydrogels and viscous polymers is added to one of the compositions with which the living cells are mixed.

23. A process as claimed in any of claims 13 to 22, wherein the setting matrix is produced by dissolving non-ceramic hydroxyapatite cement in an aqueous solution.

24. A process as claimed in any of claims 13 to 23, wherein the bone substitute material is put
into a multicomponent applicator consisting of a plurality of containers, which may also be syringes, which are combined, or
into a complete syringe with a plurality of chambers.

25. A process as claimed in any of claims 13 to 23, wherein the bone substitute material is produced in a device for preparing and administering the bone substitute material, wherein the device comprises the following features:
a) a mixing chamber with an outlet opening through which the mixture can emerge,
b) a first supply channel (main channel) leading into the mixing chamber and
c) one or more other supply channels (subsidiary channels) leading into the mixing chamber,
where the end of the subsidiary channel/the ends of the subsidiary channels are arranged in the mixing chamber so that material entering the mixing chamber from the subsidiary channel/subsidiary channels can penetrate into the material stream entering the mixing chamber from the main channel.

26. A process as claimed in claim 25, wherein in the device the internal diameter of the main channel is at least 1 mm.

27. A process as claimed in claim 25 or 26, wherein in the device the internal diameter of the subsidiary channel/subsidiary channels is not more than 1.5 mm.

28. A process as claimed in any of claims 25 to 27, wherein the device comprises 3 to 5 subsidiary channels.

29. A process as claimed in any of claims 25 to 28, wherein in the device the ends of the subsidiary channels in the mixing chamber are arranged essentially symmetrically around the end of the main channel in the mixing chamber.

30. A process as claimed in any of claims 25 to 29, wherein in the device the end of the subsidiary channel/the ends of the subsidiary channels in the mixing chamber intersect with the imaginary extension of the main channel in the mixing chamber.

31. A process as claimed in any of claims 25 to 30, wherein in the device the supply channels are connected to storage containers from which the contents of the storage containers can be delivered into the supply channels.

32. A process as claimed in claim 31, wherein in the device the storage containers are syringes.

## Revendications

1. Matériau de substitution des os, comprenant au moins les composants suivants :
a) une matrice molle qui contient de la fibrine ou du fibrinogène,
b) des cellules vivantes,
c) une matrice durcissante qui contient du ciment d'hydroxy-apatite non céramique.

2. Matériau de substitution des os suivant la revendication 1, **caractérisé en ce que** la matrice molle contient de la thrombine.

3. Matériau de substitution des os suivant la revendication 2, **caractérisé en ce que** la matrice molle contient de l'acide ε-aminocaproïque ou de l'aprotinine.

4. Matériau de substitution des os suivant une des revendications précédentes, **caractérisé en ce que** la matrice molle contient au moins une substance qui est choisie parmi le groupe comprenant du sulfate de chondroitine, des protéoglycanes, des sialoprotéines, des facteurs de croissance, des hormones et des acides nucléiques codant pour des facteurs de croissance ou des hormones.

5. Matériau de substitution des os suivant l'une des revendications précédentes, **caractérisé en ce que** la matrice molle contient au moins une substance qui est choisie parmi le groupe comprenant des gels de collagène biologiques, des gélatines, des alginates, des agaroses, des polysaccharides, du collagène synthétique, des hydrogels et des polymères visqueux.

6. Matériau de substitution des os suivant l'une des revendications précédentes, **caractérisé en ce qu'**au moins une partie importante des cellules vivantes est formée d'ostéoblastes ou de leurs cellules précurseurs.

7. Matériau de substitution des os suivant l'une des revendications précédentes, **caractérisé en ce qu'**il contient, en outre, des cellules vivantes de formation de vaisseaux.

8. Matériau de substitution des os suivant la revendication 7, **caractérisé en ce que** les cellules de formation de vaisseaux sont des cellules endothéliales ou des cellules précurseurs de celles-ci.

9. Matériau de substitution des os suivant l'une des revendications précédentes, **caractérisé en ce que** la matrice durcissante contient au moins une substance qui, par cristallisation, se combine en hydroxyapatite.

10. Matériau de substitution des os suivant l'une des revendications 1 à 9, **caractérisé en ce que** la matrice durcissante contient du PGLA.

11. Matériau de substitution des os suivant l'une des revendications précédentes, **caractérisé en ce que** la matrice durcissante se solidifie en l'espace de 15 minutes.

12. Matériau de substitution des os suivant l'une des revendications précédentes, **caractérisé en ce qu'**il est préparé
dans une seringue multiple constituée de plusieurs seringues qui sont combinées, ou
dans une seringue complète comportant plusieurs compartiments.

13. Procédé de préparation d'un matériau de substitution des os comprenant une matrice molle, des cellules vivantes et une matrice durcissante suivant l'une des revendications 1 à 12, **caractérisé en ce qu'**il présente les particularités suivantes :
a) une préparation de cellules vivantes,
b) un mélange de cellules vivantes avec une composition qui contient des éléments de formation d'une matrice molle, et
c) un mélange des cellules vivantes avec une composition qui contient une matière durcissante.

14. Procédé suivant la revendication 13, **caractérisé en ce que** l'on mélange tout d'abord les cellules vivantes avec la composition qui contient des éléments de formation d'une matrice molle, et ensuite les cellules vivantes noyées dans la matrice molle avec la composition qui contient une matière durcissante.

15. Procédé suivant l'une des revendications 13 et 14, **caractérisé en ce que** les cellules vivantes sont obtenues par une biopsie osseuse ou par aspiration de moelle osseuse.

16. Procédé suivant l'une des revendications 13 à 15 **caractérisé en ce que** les cellules vivantes sont cultivées in vitro avant l'étape b) et c).

17. Procédé suivant l'une des revendications 13 à 16, **caractérisé en ce qu'**au moins une partie des cellules vivantes sont des ostéoblastes ou des cellules précurseurs de ceux-ci.

18. Procédé suivant l'une des revendications 13 à 17, **caractérisé en ce que** la matrice molle est préparée par mise en contact d'une solution de fibrinogène et d'une solution de thrombine.

19. Procédé suivant la revendication 18, **caractérisé en ce que** le fibrinogène est tout d'abord dissous dans un milieu d'ostéoblastes ou une solution saline physiologique (0,9% de NaCl) ou une solution saline tamponnée au phosphate (PBS).

20. Procédé suivant l'une des revendications 18 et 19, **caractérisé en ce que** la solution de fibrinogène est stabilisée par de l'acide ε-aminocaproïque.

21. Procédé suivant l'une des revendications 13 à 20, **caractérisé en que**, à une des compositions avec lesquelles les cellules vivantes sont mélangées, on ajoute au moins une substance qui est choisie parmi le groupe comprenant du sulfate de chondroitine, des protéoglycanes, des sialoprotéines, des facteurs de croissance, des hormones et des acides nucléiques codant pour des facteurs de croissance ou des hormones.

22. Procédé suivant l'une des revendications 13 à 21, **caractérisé en ce que**, à une des compositions avec lesquelles les cellules vivantes sont mélangées, on ajoute au moins une substance qui est choisie parmi le groupe comprenant des gels de collagène biologiques, des gélatines, des alginates, des agaroses, des polysaccharides, du collagène synthétique, des hydrogels et des polymères visqueux.

23. Procédé suivant l'une des revendications 13 à 22, **caractérisé en ce que** la matrice durcissante est préparée par dissolution de ciment d'hydroxyapatite non céramique dans une solution aqueuse.

24. Procédé suivant l'une des revendications 13 à 23, **caractérisé en ce que** le matériau de substitution des os est versé
dans un applicateur à plusieurs composants, constitué de plusieurs conteneurs qui peuvent aussi être des seringues qui sont combinées, ou
dans une seringue complète comportant plusieurs compartiments.

25. Procédé suivant l'une des revendications 13 à 23, **caractérisé en ce que** le matériau de substitution des os est préparé dans un dispositif de préparation et d'administration du matériau de substitution des os, le dispositif présentant les particularités suivantes :
a) un compartiment de mélange avec une ouverture de sortie par laquelle le mélange peut sortir,
b) un premier canal d'admission menant dans le compartiment de mélange (canal principal), et
c) un ou plusieurs autres canaux d'admission menant dans le compartiment de mélange (canaux secondaires),
la ou les extrémités du ou des canaux secondaires étant agencées dans le compartiment de mélange de façon que de la matière entrant dans le compartiment de mélange à partir du ou des canaux secondaires puisse pénétrer dans le courant de matière entrant dans le compartiment de mélange à partir du canal principal.

26. Procédé suivant la revendication 25, **caractérisé en ce que**, dans le dispositif, le diamètre interne du canal principal est d'au moins 1 mm.

27. Procédé suivant l'une des revendications 25 et 26, **caractérisé en ce que**, dans le dispositif, le diamètre interne du ou des canaux secondaires est d'au maximum de 1,5 mm.

28. Procédé suivant l'une des revendications 25 à 27, **caractérisé en ce que** le dispositif comporte 3 à 5 canaux secondaires.

29. Procédé suivant l'une des revendications 25 à 28, **caractérisé en ce que**, dans le dispositif, les extrémités des canaux secondaires dans le compartiment de mélange sont agencées sensiblement symétriquement autour de l'extrémité du canal principal, dans le compartiment de mélange.

30. Procédé suivant l'une des revendications 25 à 29, **caractérisé en ce que**, dans le dispositif, la ou les extrémités du ou des canaux secondaires dans le compartiment de mélange croisent la prolongation imaginaire du canal principal dans le compartiment de mélange.

31. Procédé suivant l'une des revendications 25 à 30, **caractérisé en ce que**, dans le dispositif, des récipients de réserve, à partir desquels le contenu des récipients de réserve peut être amené dans les canaux d'admission, sont en communication avec les canaux d'admission.

32. Procédé suivant la revendication 31, **caractérisé en ce que**, dans le dispositif, les récipients de réserve sont des seringues.
